# EUROPEAN PATENT APPLICATION

(11) **EP 4 321 158 A1**
(43) Date of publication of application: **14.02.2024**
(21) Application number: 22750757.1
(22) Date of filing: 08.04.2022
(51) Int. Cl.: A61K 31/352, A61K 9/50, A61K 9/127, A61P 31/12, A61P 31/14, A23K 20/121, A23K 20/195, A61K 31/675

(54) **PHARMACEUTICAL COMPOSITION FOR PREVENTING OR TREATING VIRAL INFECTIONS, CONTAINING ROTTLERIN AS ACTIVE INGREDIENT**

(30) Priority: 08.04.2021 KR 20210046145; 15.07.2021 KR 20210093159
(71) Applicant: Qvet, Gwangjin-gu Seoul 05066 (KR)
(72) Inventor: SONG, Juyeon, Namyangju-si Gyeonggi-do 12168 (KR)
(74) Representative: J A Kemp LLP
(86) International application number: PCT/KR2022/005141
(87) International publication number: WO 2022/216118

(57) **Abstract**

The present disclosure relates to a pharmaceutical composition comprising rottlerin as an active ingredient for the prevention or treatment of a virus infection. The pharmaceutical composition of the present disclosure for the prevention or treatment of a virus infection comprising rottlerin as an active ingredient can be advantageously used to treat a virus infection caused by coronavirus, porcine epidemic diarrhea virus, transmissible gastroenteritis virus, porcine circovirus type 2, porcine reproductive and respiratory syndrome virus, or the like.

## Description

### Technical Field

This application claims priority to and the benefit of Korean Patent Application No. 10-2021-0046145 filed in the Korean Intellectual Property Office on 8 April 2021 and Korean Patent Application No. 10-2021-0093159 filed in the Korean Intellectual Property Office on 15 July 2021, the disclosures of which are incorporated herein by reference.

The present disclosure relates to a pharmaceutical composition for prevention or treatment of a virus infection, the pharmaceutical composition comprising rottlerin as an active ingredient.

### Background Art

Porcine reproductive and respiratory syndrome (PRRS) was first observed in 1986 in the United States and in 1990 in Europe. In 1991, Dr. Wensvoort at the Netherlands Central Veterinary Laboratory isolated a causative virus for the disease by using porcine alveolar macrophage for the first time and thus revealed the disease to be caused by a virus (PRRSV). This virus was named Lelystad virus after the place name where the corresponding laboratory was located. Thereafter, the Lelystad virus was called by names, such as swine infertility and respiratory syndrome (SIRS) virus, porcine epidemic abortions and respiratory syndrome (PEARS) virus, blue-eared pig disease virus, and the like, but was internationally designated as porcine reproductive and respiratory syndrome (PRRS) virus in 1992. It is known that when having porcine reproductive and respiratory syndrome by the virus, piglets and rearing pigs show respiratory symptoms, such as cough, dyspnea, and pneumonia; sows show abortion or stillbirth in the late stage of pregnancy, premature farrowing or deliver weak piglets; and boars have abnormality in semen properties, and therefore, the syndrome, as its name, is characterized by reproductive disorders and respiratory symptom-causing poor growth. Attenuated vaccines and killed vaccines are known as vaccines for preventing porcine reproductive and respiratory syndrome, but the attenuated vaccines have a drawback of causing diseases and the killed vaccines have economic problems that the production cost is high and each individual needs to be vaccinated. Moreover, an appropriate medicine for porcine reproductive and respiratory syndrome is not known since the PRRS virus replicates and multiplies in cells.

Porcine epidemic diarrhea (PED) causes acute enteritis and watery diarrhea in piglets and shows a mortality rate of 100% in young piglets. This disease is one of the representative pig diseases that cause enormous economic damage to the pig industry after the first occurrence thereof was reported in Belgium and England in the mid-1970s. The disease occurred in Asian countries, such as China and Japan since the 1990s, and has been continuously occurring after it was first reported in 1992 in Korea. Since 2007, the disease has also occurred in Southeast Asian countries, such as Thailand, Vietnam, and the Philippines, causing economic damage to many pig farms. PED mainly occurred in Asian countries and some European countries before 2013, but since May 2013, the disease first occurred as acute PED in the United States and spread throughout the United States, with a high mortality rate in infected piglets. As such, PED viruses are highly pathogenic and cause economic damage to the pig industry, and thus the development of appropriate vaccines is needed and appropriate medicines are also needed.

Feline coronavirus (FCoV) belongs to the Coronaviridae family, a group of enveloped, positive strand RNA viruses, commonly found in cats. In nature, FCoV exists as two distinct biotypes: feline enteric coronavirus (FECV) and feline infectious peritoneal virus (FIPV) which is a mutated form of FECV. The FECV infection is widespread in cats, and 40-80% of cats are estimated to shed viruses worldwide. FECV chronically infects gastrointestinal epithelial cells in cats and is typically transmitted through the fecal-oral route. The FECV infection in cats is largely asymptomatic, with some cats experiencing diarrhea, vomiting, loss of appetite, and fever. The FIPV biotype occurs following a single nucleotide polymorphism or deletion that inactivates the viral 3c protease gene in FECV, but is also implicated with mutations within the viral spike protein. The inactivation of the 3c protease alters cell tropism to enable the virus to replicate within macrophages, facilitating systemic dissemination of FIPV and the onset of feline infectious peritonitis (FIP). FIP is a progressive, immune-related disease in cats. The FIP disease can take the form of "wet" or "dry" FIP. Wet FIP is associated with an inflammation of the visceral serosa and omentum resulting in the exudation of fluids into the abdomen and/or chest cavities. Dry FIP is characterized by granulomatous involvement of paracnchymatous organs such as the liver, central nervous system, or eyes. The development of either the wet or dry form of FIP is invariably fatal. FIP is a major problem in environments with large cat densities such as, multi-cat households, catteries, shelters, and cat rescue facilities. The disease is most prevalent in younger cats (<3 years old), and particularly kittens, due to the higher levels of replicating FECV, which increases the likelihood of mutation to the FIPV biotype, as well as reduced resistance to viruses harboring those mutations. FIP is a leading cause of death in cats under 2 years of age and is estimated to kill between 0.3 and 1% of cats worldwide. Currently, there are no approved vaccines or effective antiviral therapies for the treatment of FIP. Therefore, there is a need for the development of agents to treat FIP in cats.

Porcine circovirus type 2 (PCV2) is a small (17-22 nm in diameter), icosahedral, non-enveloped DNA virus, which contains a single-stranded circular genome. PCV2 shares approximately 80% sequence identity with porcine circovirus type 1 (PCV-1). However, in contrast with PCV1, which is generally non-virulent, pigs infected with PCV2 typically shows a syndrome called post-weaning multi-systemic wasting syndrome (PMWS). PMWS is clinically characterized by wasting, paleness of the skin, unthriftiness, respiratory distress, diarrhea, icterus, and jaundice. In some affected pigs, a combination of all symptoms will be apparent while other affected pigs will only have one or two of these symptoms. During necropsy, microscopic and macroscopic lesions also appear on multiple tissues and organs, with lymphoid organs being the most common site for lesions. A strong correlation has been observed between the amount of PCV2 nucleic acid or antigen and the severity of microscopic lymphoid lesions. Mortality rates for pigs infected with PCV2 can approach 80%. In addition to PMWS, PCV2 has been associated with several other infections including pseudorabies, porcine reproductive and respiratory syndrome (PRRS), Glasser's disease, streptococcal meningitis, salmonellosis, postweaning colibacillosis, dietetic hepatosis, and suppurative bronchopneumonia. A PCV2a genotype-based vaccine has been currently released for the prevention of PCV2, but there is no antiviral therapy for PCV2. Therefore, there is a need for the development of agents to treat PCV2.

Throughout the entire specification, many patent documents are referenced and their citations are represented. The disclosures of cited papers and patent documents are entirely incorporated by reference into the present specification, and the level of the technical field within which the present disclosure falls and details of the present disclosure are explained more clearly.

### Disclosure of Invention

### Technical Problem

The present inventors have endeavored to develop a therapeutic agent capable of effectively acting on viruses. As a result, the present inventors established that a composition comprising rottlerin exerts an excellent antiviral effect on several types of viruses, and thus completed the present disclosure.

Accordingly, an aspect of the present disclosure is to provide a pharmaceutical composition for the prevention or treatment of a virus infection, the pharmaceutical composition comprising rottlerin as an active ingredient.

Another aspect of the present disclosure is to provide a food composition for the prevention or alleviation of a virus infection, the food composition comprising rottlerin.

Still another aspect of the present disclosure is to provide a method for treating a virus infection, the method comprising administering the pharmaceutical composition to a subject.

Still another aspect of the present disclosure is to provide a method for alleviating a virus infection, the method comprising administering the food composition to a subject.

Other purposes and advantages of the present disclosure will become more obvious when taken with the following detailed description of the invention, claims, and drawings.

### Solution to Problem

In accordance with an aspect of the present disclosure, there is provided a pharmaceutical composition for prevention or treatment of a virus infection, the pharmaceutical composition comprising rottlerin as an active ingredient.

Rottlerin is a compound separated from a natural polyphenol extract isolated and extracted from the tree *Mallotus philippensis.* Natural products containing the rottlerin compound may be extracted by the following methods, but are not limited thereto. The natural products may be used by extraction through extraction methods that are widely known in the art or by purchase.

A powder of *Mallotus philippensis* fruits is subjected to reaction for 3-9 hours while immersed in 70-99% ethyl alcohol, and then filtered through a filter. Rottlerin is not dissolved in ethyl alcohol, and thus the residue that has not passed through the filter is dried, and then extracted 2-6 times by continuous stirring with ethyl acetate for 2-8 hours. The residue remaining after 2 to 6 times of extraction was washed with 70-99% alcohol at 1-7°C and crystallized with ethyl acetate. The crystals manufactured by the above method contain rottlerin as a marker component.

In an embodiment of the present disclosure, the rottlerin may be rottlerin crystals. The rottlerin crystals is in the form of microparticles.

In an embodiment of the present disclosure, the microparticles containing rottlerin are obtained by sonicating an extract comprising rottlerin or crystals of a rottlerin compound.

In an embodiment of the present disclosure, the extract comprising rottlerin or the crystals of a rottlerin compound have a rottlerin purity of at least 90%, 91%, 92%, 93%, 94%, 95%, 96%, 97%, 98%, or 99%.

In an embodiment of the present disclosure, the microparticles containing rottlerin have a particle size of 200 nm or less. More specifically, the microparticles containing rottlerin have a particle size of 10 nm to 200 nm, 20 nm to 200 nm, 30 nm to 200 nm, 40 nm to 200 nm, 50 nm to 200 nm, 60 nm to 200 nm, 70 nm to 200 nm, 80 nm to 200 nm, 90 nm to 200 nm, 100 nm to 200 nm, 10 nm to 150 nm, 20 nm to 150 nm, 30 nm to 150 nm, 40 nm to 150 nm, 50 nm to 150 nm, 60 nm to 150 nm, 70 nm to 150 nm, 80 nm to 150 nm, 90 nm to 150 nm, 100 nm to 150 nm, 10 nm to 125 nm, 20 nm to 125 nm, 30 nm to 125 nm, 40 nm to 125 nm, 50 nm to 125 nm, 60 nm to 125 nm, 70 nm to 125 nm, 80 nm to 125 nm, 90 nm to 125 nm, or 100 nm to 125 nm.

In an embodiment of the present disclosure, the rottlerin may be contained in liposomes.

As used herein, the term liposome refers to a phospholipid bilayer capable of encapsulating an active drug therein.

In an embodiment of the present disclosure, the size of the liposome is 1 to 500 nm. More specifically, the size of the liposome is 1 to 300 nm, 1 to 200 nm, 1 to 150 nm, 1 to 120 nm, 1 to 100 nm, 1 to 90 nm, 1 to 80 nm, 1 to 70 nm, 10 to 500 nm, 10 to 300 nm, 10 to 200 nm, 10 to 150 nm, 10 to 120 nm, 10 to 100 nm, 10 to 90 nm, 10 to 80 nm, 10 to 70 nm, 30 to 500 nm, 30 to 300 nm, 30 to 200 nm, 30 to 150 nm, 30 to 120 nm, 30 to 100 nm, 30 to 90 nm, 30 to 80 nm, 30 to 70 nm, 50 to 500 nm, 50 to 300 nm, 50 to 200 nm, 50 to 150 nm, 50 to 120 nm, 50 to 100 nm, 50 to 90 nm, 50 to 80 nm, 50 to 70 nm, 60 to 500 nm, 60 to 300 nm, 60 to 200 nm, 60 to 150 nm, 60 to 120 nm, 60 to 100 nm, 60 to 90 nm, 60 to 80 nm, 60 to 70 nm, 61 to 70 nm, 63 to 70 nm, 64 to 70 nm, 60 to 69 nm, 61 to 69 nm, 63 to 69 nm, 64 to 69 nm, 60 to 68 nm, 61 to 68 nm, 63 to 68 nm, 64 to 68 nm, or 65 to 67 nm.

In an embodiment of the present disclosure, the liposome comprises a phosphatidylcholine and a sterol-based compound.

In an embodiment of the present disclosure, the phosphatidylcholine and the sterol-based compound have a molar ratio of 0.1-15:1. More specifically, the molar ratio of the phosphatidylcholine and the sterol-based compound is 0.1-15:1, 0.2-15:1, 0.3-15:1, 0.4-15:1, 0.5-15:1, 0.6-15:1, 0.7-15:1, 0.8-15:1, 0.9-15:1, 1-15:1, 1.1-15:1, 1.2-15:1, 1.3-15:1, 1.4-15:1, 1.5-15:1, 1.6-15:1, 1.7-15:1, 1.8-15:1, 1.9-15:1, 2-15:1, 2.1-15:1, 2.2-15:1, 2.3-15:1, 2.4-15:1, 2.5-15:1, 2.6-15:1, 2.7-15:1, 2.8-15:1, 2.9-15:1, 3-15:1, 3.5-15:1, 4-15:1, 4.5-15:1, 5-15:1, 6-15:1, 7-15:1, 8-15:1, 9-15:1, 10-15:1, 11-15:1, 12-15:1, 13-15:1, 14-15:1, 0.1-12:1, 0.2-12:1, 0.3-12:1, 0.4-12:1, 0.5-12:1, 0.6-12:1, 0.7-12:1, 0.8-12:1, 0.9-12:1, 1-12:1, 1.1-12:1, 1.2-12:1, 1.3-12:1, 1.4-12:1, 1.5-12:1, 1.6-12:1, 1.7-12:1, 1.8-12:1, 1.9-12:1, 2-12:1, 2.1-12:1, 2.2-12:1, 2.3-12:1, 2.4-12:1, 2.5-12:1, 2.6-12:1, 2.7-12:1, 2.8-12:1, 2.9-12:1, 3-12:1, 3.5-12:1, 4-12:1, 4.5-12:1, 5-12:1, 6-12:1, 7-12:1, 8-12:1, 9-12:1, 10-12:1, 11-12:1, 0.1-10:1, 0.2-10:1, 0.3-10:1, 0.4-10:1, 0.5-10:1, 0.6-10:1, 0.7-10:1, 0.8-10:1, 0.9-10:1, 1-10:1, 1.1-10:1, 1.2-10:1, 1.3-10:1, 1.4-10:1, 1.5-10:1, 1.6-10:1, 1.7-10:1, 1.8-10:1, 1.9-10:1, 2-10:1, 2.1-10:1, 2.2-10:1, 2.3-10:1, 2.4-10:1, 2.5-10:1, 2.6-10:1, 2.7-10:1, 2.8-10:1, 2.9-10:1, 3-10:1, 3.5-10:1, 4-10:1, 4.5-10:1, 5-10:1, 6-10:1, 7-10:1, 8-10:1, 9-10:1, 0.1-8:1, 0.2-8:1, 0.3-8:1, 0.4-8:1, 0.5-8:1, 0.6-8:1, 0.7-8:1, 0.8-8:1, 0.9-8:1, 1-8:1, 1.1-8:1, 1.2-8:1, 1.3-8:1, 1.4-8:1, 1.5-8:1, 1.6-8:1, 1.7-8:1, 1.8-8:1, 1.9-8:1, 2-8:1, 2.1-8:1, 2.2-8:1, 2.3-8:1, 2.4-8:1, 2.5-8:1, 2.6-8:1, 2.7-8:1, 2.8-8:1, 2.9-8:1, 3-8:1, 3.5-8:1, 4-8:1, 4.5-8:1, 5-8:1, 6-8:1, 7-8:1, 0.1-7:1, 0.2-7:1, 0.3-7:1, 0.4-7:1, 0.5-7:1, 0.6-7:1, 0.7-7:1, 0.8-7:1, 0.9-7:1, 1-7:1, 1.1-7:1, 1.2-7:1, 1.3-7:1, 1.4-7:1, 1.5-7:1, 1.6-7:1, 1.7-7:1, 1.8-7:1, 1.9-7:1, 2-7:1, 2.1-7:1, 2.2-7:1, 2.3-7:1, 2.4-7:1, 2.5-7:1, 2.6-7:1, 2.7-7:1, 2.8-7:1, 2.9-7:1, 3-7:1, 3.5-7:1, 4-7:1, 4.5-7:1, 5-7:1, 6-7:1, 0.1-6:1, 0.2-6:1, 0.3-6:1, 0.4-6:1, 0.5-6:1, 0.6-6:1, 0.7-6:1, 0.8-6:1, 0.9-6:1, 1-6:1, 1.1-6:1, 1.2-6:1, 1.3-6:1, 1.4-6:1, 1.5-6:1, 1.6-6:1, 1.7-6:1, 1.8-6:1, 1.9-6:1, 2-6:1, 2.1-6:1, 2.2-6:1, 2.3-6:1, 2.4-6:1, 2.5-6:1, 2.6-6:1, 2.7-6:1, 2.8-6:1, 2.9-6:1, 3-6:1, 3.5-6:1, 4-6:1, 4.5-6:1, 5-6:1, 0.1-5:1, 0.2-5:1, 0.3-5:1, 0.4-5:1, 0.5-5:1, 0.6-5:1, 0.7-5:1, 0.8-5:1, 0.9-5:1, 1-5:1, 1.1-5:1, 1.2-5:1, 1.3-5:1, 1.4-5:1, 1.5-5:1, 1.6-5:1, 1.7-5:1, 1.8-5:1, 1.9-5:1, 2-5:1, 2.1-5:1, 2.2-5:1, 2.3-5:1, 2.4-5:1, 2.5-5:1, 2.6-5:1, 2.7-5:1, 2.8-5:1, 2.9-5:1, 3-5:1, 3.5-5:1, 4-5:1, 4.5-5:1, 0.1:1, 0.2:1, 0.3:1, 0.4:1, 0.5:1, 0.6:1, 0.7:1, 0.8:1, 0.9:1, 1:1, 1.1:1, 1.2:1, 1.3:1, 1.4:1, 1.5:1, 1.6:1, 1.7:1, 1.8:1, 1.9:1, 2:1, 2.1:1, 2.2:1, 2.3:1, 2.4:1, 2.5:1, 2.6:1, 2.7:1, 2.8:1, 2.9:1, 3:1, 3.5:1, 4:1, 4.5:1, 5:1, 6:1, 7:1, 8:1, 9:1, 10:1, 11:1, 12:1, 13:1, 14:1, or 15:1.

In an embodiment of the present disclosure, the lipid comprising the phosphatidylcholine and the sterol-based compound and rottlerin have a weight ratio of 0.1-150:1. More specifically, the weight ratio of the lipid and rottlerin is 0.1-150:1, 0.2-150:1, 0.3-150:1, 0.4-150:1, 0.5-150:1, 0.6-150:1, 0.7-150:1, 0.8-150:1, 0.9-150:1, 1-150:1, 1.5-150:1, 2-150:1, 3-150:1, 4-150:1, 5-150:1, 6-150:1, 7-150:1, 8-150:1, 9-150:1, 10-150:1, 15-150:1, 20-150:1, 25-150:1, 30-150:1, 35-150:1, 40-150:1, 45-150:1, 50-150:1, 55-150:1, 60-150:1, 70-150:1, 80-150:1, 90-150:1, 95-150:1, 100-150:1, 110-150:1, 115-150:1, 120-150:1, 125-150:1, 130-150:1, 135-150:1, 140-150:1, 145-150:1, 0.1-140:1, 0.2-140:1, 0.3-140:1, 0.4-140:1, 0.5-140:1, 0.6-140:1, 0.7-140:1, 0.8-140:1, 0.9-140:1, 1-140:1, 1.5-140:1, 2-140:1, 3-140:1, 4-140:1, 5-140:1, 6-140:1, 7-140:1, 8-140:1, 9-140:1, 10-140:1, 15-140:1, 20-140:1, 25-140:1, 30-140:1, 35-140:1, 40-140:1, 45-140:1, 50-140:1, 55-140:1, 60-140:1, 70-140:1, 80-140:1, 90-140:1, 95-140:1, 100-140:1, 110-140:1, 115-140:1, 120-140:1, 125-140:1, 130-140:1, 135-140:1, 0.1-130:1, 0.2-130:1, 0.3-130:1, 0.4-130:1, 0.5-130:1, 0.6-130:1, 0.7-130:1, 0.8-130:1, 0.9-130:1, 1-130:1, 1.5-130:1, 2-130:1, 3-130:1, 4-130:1, 5-130:1, 6-130:1, 7-130:1, 8-130:1, 9-130:1, 10-130:1, 15-130:1, 20-130:1, 25-130:1, 30-130:1, 35-130:1, 40-130:1, 45-130:1, 50-130:1, 55-130:1, 60-130:1, 70-130:1, 80-130:1, 90-130:1, 95-130:1, 100-130:1, 110-130:1, 115-130:1, 120-130:1, 125-130:1, 0.1-120:1, 0.2-120:1, 0.3-120:1, 0.4-120:1, 0.5-120:1, 0.6-120:1, 0.7-120:1, 0.8-120:1, 0.9-120:1, 1-120:1, 1.5-120:1, 2-120:1, 3-120:1, 4-120:1, 5-120:1, 6-120:1, 7-120:1, 8-120:1, 9-120:1, 10-120:1, 15-120:1, 20-120:1, 25-120:1, 30-120:1, 35-120:1, 40-120:1, 45-120:1, 50-120:1, 55-120:1, 60-120:1, 70-120:1, 80-120:1, 90-120:1, 95-120:1, 100-120:1, 110-120:1, 115-120:1, 0.1-110:1, 0.2-110:1, 0.3-110:1, 0.4-110:1, 0.5-110:1, 0.6-110:1, 0.7-110:1, 0.8-110:1, 0.9-110:1, 1-110:1, 1.5-110:1, 2-110:1, 3-110:1, 4-110:1, 5-110:1, 6-110:1, 7-110:1, 8-110:1, 9-110:1, 10-110:1, 15-110:1, 20-110:1, 25-110:1, 30-110:1, 35-110:1, 40-110:1, 45-110:1, 50-110:1, 55-110:1, 60-110:1, 70-110:1, 80-110:1, 90-110:1, 95-110:1, 100-110:1, 0.1-105:1, 0.2-105:1, 0.3-105:1, 0.4-105:1, 0.5-105:1, 0.6-105:1, 0.7-105:1, 0.8-105:1, 0.9-105:1, 1-105:1, 1.5-105:1, 2-105:1, 3-105:1, 4-105:1, 5-105:1, 6-105:1, 7-105:1, 8-105:1, 9-105:1, 10-105:1, 15-105:1, 20-105:1, 25-105:1, 30-105:1, 35-105:1, 40-105:1, 45-105:1, 50-105:1, 55-105:1, 60-105:1, 70-105:1, 80-105:1, 90-105:1, 95-105:1, 100-105:1, 0.1-100:1, 0.2-100:1, 0.3-100:1, 0.4-100:1, 0.5-100:1, 0.6-100:1, 0.7-100:1, 0.8-100:1, 0.9-100:1, 1-100:1, 1.5-100:1, 2-100:1, 3-100:1, 4-100:1, 5-100:1, 6-100:1, 7-100:1, 8-100:1, 9-100:1, 10-100:1, 15-100:1, 20-100:1, 25-100:1, 30-100:1, 35-100:1, 40-100:1, 45-100:1, 50-100:1, 55-100:1, 60-100:1, 70-100:1, 80-100:1, 90-100:1, 95-100:1, 0.1-95:1, 0.2-95:1, 0.3-95:1, 0.4-95:1, 0.5-95:1, 0.6-95:1, 0.7-95:1, 0.8-95:1, 0.9-95:1, 1-95:1, 1.5-95:1, 2-95:1, 3-95:1, 4-95:1, 5-95:1, 6-95:1, 7-95:1, 8-95:1, 9-95:1, 10-95:1, 15-95:1, 20-95:1, 25-95:1, 30-95:1, 35-95:1, 40-95:1, 45-95:1, 50-95:1, 55-95:1, 60-95:1, 70-95:1, 80-95:1, or 90-95:1, but is not limited thereto.

In an embodiment of the present disclosure, the phosphatidylcholine is selected from the group consisting of hydrogenated soy phosphatidylcholine (HSPC), 1,2-dimyristoyl-sn-glycero-3-phosphocholine (DMPC), 1,2-dihexanoyl-sn-glycero-3-phosphocholine (DHPC), 1,2-diheptanoyl-sn-glycero-3-phosphocholine, 1,2-dioctanoyl-sn-glycero-3-phosphocholine, 1,2-dinonanoyl-sn-glycero-3-phosphocholine, 1,2-didecanoyl-sn-glycero-3-phosphocholine, 1,2-diundecanoyl-sn-glycero-3-phosphocholine, 1,2-dilauroyl-sn-glycero-3-phosphocholine (DLPC), 1,2-ditridecanoyl-sn-glycero-3-phosphocholine, 1,2-dipentadecanoyl-sn-glycero-3-phosphocholine, 1,2-dipalmitoyl-sn-glycero-3-phosphocholine (DPPC), 1,2-diheptadecanoyl-sn-glycero-3-phosphocholine, and 1,2-distearoyl-sn-glycero-3-phosphocholine (DSPC).

According to an embodiment of the present disclosure, the phosphatidylcholine is hydrogenated soy phosphatidylcholine (HSPC).

In an embodiment of the present disclosure, the sterol-based compound is selected from the group consisting of cholesterol, 3b-[N-(N',N'-dimethylaminoethane)-cabamyl]cholesterol (DC-Chol), stigmasterol, campesterol, sitosterol, ergosterol, lanosterol, dinosterol, gorgosterol, avenasterol, saringosterol, fucosterol, cholesteryl hemisuccinate, cholesteryl benzoate, cholesteryl oleate, cholesteryl oleyl carbonate, cholesteryl isostearate, cholesteryl linoleate, cholesteryl acetate, cholesteryl palmitate, cholesteryl stearate, cholesteryl chloride, cholesteryl nonanoate, and cholesteryl arachidonate.

In an embodiment of the present disclosure, the virus is a virus belonging to a family selected from the group consisting of the families Bunyaviridae, Arteriviridae, Coronaviridae, Filoviridae, Flaviviridae, Hepadnaviridae, Orthomyxoviridae, Poxviridae, Rhabdoviridae, Retroviridae, Togaviridae, and Herpesviridae.

More specifically, the virus may be: Sin Nombre Hantavirus or the like belonging to the family Bunyaviridae; porcine reproductive and respiratory syndrome virus or the like belonging to the family Arteriviridae; coronavirus involved in various acute respiratory and diarrhea syndromes or the like belonging to the family Coronaviridae; PCV2 virus or the like belonging to the family Circoviridae; Ebola virus, Marburg virus, or the like belonging to the family Filoviridae; West Nile virus, yellow fever virus, Dengue fever virus, Hepatitis C virus, or the like belonging to the family Flaviviridae; Hepatitis B virus or the like belonging to the family Hepadnaviridae; influenza virus or the like belonging to the family Orthomyxoviridae; Smallpox virus, vaccinia virus, Molluscum contagiosum virus, monkeypox virus, or the like belonging to the family Poxviridae; vesicular stomatitis virus or the like belonging to the family Rhabdoviridae; human immunodeficiency virus (HIV) or the like belonging to the family Retroviridae; Chikungunya virus or the like belonging to the family Togaviridae; herpes simplex 1 virus, herpes simplex 2 virus, pseudorabies virus, human herpes virus(HHV), or the like belonging to the family Herpesviridae; or the like.

In an embodiment of the present disclosure, the virus infection is a disease caused by a virus entering an animal or human body and multiplying in an organ or tissue, and an example thereof is: nephrotic hemorrhagic fever (epidemic hemorrhagic fever) caused by infection of a virus belonging to the family Bunyaviridae; porcine reproductive and respiratory syndrome caused by infection of a virus of the family Arteriviridae; a respiratory disease such as sinus cold, transmissible gastroenteritis (TGE), or porcine epidemic diarrhea (PED), which is caused by infection of a virus belonging to the family Coronaviridae; hepatitis C caused by infection of a virus belonging to the family Flaviviridae; hepatitis B caused by infection of a virus belonging to the family Hepadnaviridae; a porcine circovirus infection caused by infection of a virus belonging to the family Circoviridae; herpes zoster caused by infection of a virus belonging to the family Herpesviridae; a flu or an influenza virus infection caused by infection of a virus belonging to the family Orthomyxoviridae; smallpox caused by infection of a virus belonging to the family Poxviridae; rabies or vesicular stomatitis caused by infection of a virus belonging to the family Rhabdoviridae; acquired immunodeficiency syndrome caused by infection of a virus belonging to the family Retroviridae; or the like, and another example thereof is a flu or an influenza virus infection caused by infection of an influenza virus belong to the family Orthomyxoviridae.

In an embodiment of the present disclosure, the virus infection is selected from the group consisting of porcine reproductive and respiratory syndrome, porcine epidemic diarrhea, transmissible gastroenteritis, a porcine circovirus type 2 infection, and a porcine respiratory coronavirus infection.

In an embodiment of the present disclosure, the virus is porcine reproductive and respiratory syndrome or porcine epidemic diarrhea.

As used herein, the term "porcine reproductive and respiratory syndrome" refers to a disease caused by porcine reproductive and respiratory syndrome virus, which is a positive-strand RNA virus and belongs to Arteriviridae. The porcine reproductive and respiratory syndrome virus has the RNA genome of about 15 kb and encodes nine open reading frames (OFRs). The major structure proteins are GP5, M, and N encoded by ORFs 5, 6, and 7. The GP5 protein is a 25-kDa envelope protein and induces strong neutralizing ability. The M protein is an 18-kDa matrix protein and induces a cellular immune response.

As used herein, the term "coronavirus" refers to a virus, which is an RNA virus belonging to the subfamily Coronavirinae in the family Coronaviridae and causes respiratory and digestive system infections in humans and animals. Spike proteins of the coronavirus determines host and tissue tropism and invade cells by binding to the host cell membrane through a receptor-binding protein.

In an embodiment of the present disclosure, the coronavirus is a virus selected from the group consisting of porcine epidemic diarrhea virus (PEDV), transmissible gastroenteritis virus (TGEV), porcine hemagglutinating encephalomyelitis virus (PHEV), porcine deltacoronavirus (PDCoV), canine coronavirus (CCoV), feline coronavirus (FCoV) or feline infectious peritonitis, bovine coronavirus (BCoV), equine coronavirus (EqCoV), murine coronavirus (MuCoV), and avian infectious bronchitis virus (IBV), but is not limited thereto.

In an embodiment of the present disclosure, the virus is porcine circovirus type 2.

The pharmaceutical composition of the present disclosure may comprise a pharmaceutically acceptable carrier in addition to the composition which is an active ingredient.

The pharmaceutically acceptable carrier contained in the pharmaceutical composition of the present disclosure is ordinarily used at the time of formulation, and examples thereof may include, but are not limited to, lactose, dextrose, sucrose, maltose, sorbitol, mannitol, starch, acacia gum, calcium phosphate, alginate, gelatin, calcium silicate, microcrystalline cellulose, polyvinylpyrrolidone, cellulose, water, syrup, methyl cellulose, methyl hydroxybenzoate, propyl hydroxybenzoate, talc, magnesium stearate, and mineral oils.

The pharmaceutical composition of the present disclosure may further comprise, in addition to the above ingredients, a lubricant, a wetting agent, a sweetening agent, a flavoring agent, an emulsifier, a suspending agent, a preservative, and the like. Appropriate pharmaceutically acceptable carriers and agents are described in detail in Remington's Pharmaceutical Sciences (19th ed., 1995).

The pharmaceutical composition of the present disclosure may be administered orally or parenterally, and examples of the administration may be intrathecal administration, intravenous administration, subcutaneous administration, intradermal administration, intramuscular administration, intraperitoneal administration, intrasternal administration, intratumoral injection, intranasal administration, intracerebral administration, intracranial administration, intrapulmonary administration, and rectal administration, but are not limited thereto.

In an embodiment of the present disclosure, the pharmaceutical composition is intranasally administered.

The appropriate dose of the pharmaceutical composition of the present disclosure varies depending on factors, such as a formulating method, a manner of administration, age, body weight, sex, and morbidity of a subject, a food, a time of administration, a route of administration, an excretion rate, and response sensitivity, and ordinarily skilled veterinarians and physicians can easily determine and prescribe the dose (pharmaceutically effective amount) that is effective for desired treatment or prevention.

In an embodiment of the present disclosure, the dose of the pharmaceutical composition is 0.0001-100 mg/kg.

More specifically, the dose of the pharmaceutical composition is 0.001-100 mg/kg, 0.005-100 mg/kg, 0.01-100 mg/kg, 0.1-100 mg/kg, 1-100 mg/kg, 5-100 mg/kg, 10-100 mg/kg, 0.001-50 mg/kg, 0.005-50 mg/kg, 0.01-50 mg/kg, 0.1-50 mg/kg, 1-50 mg/kg, 5-50 mg/kg, 10-50 mg/kg, 0.001-10 mg/kg, 0.005-10 mg/kg, 0.01-10 mg/kg, 0.1-10 mg/kg, 1-10 mg/kg, 5-10 mg/kg, 0.001-1 mg/kg, 0.005-1 mg/kg, 0.01-1 mg/kg, 0.1-1 mg/kg, 0.001-0.5 mg/kg, 0.005-0.5 mg/kg, 0.01-0.5 mg/kg, 0.1-0.5 mg/kg, 0.001-0.1 mg/kg, 0.005-0.1 mg/kg, 0.01-0.1 mg/kg, 0.001-0.05 mg/kg, 0.005-0.05 mg/kg, or 0.01-0.05 mg/kg, but is not limited thereto.

As used herein, the term "pharmaceutically effective amount" refers to an amount sufficient to prevent or treat the above-described diseases.

As used herein, the term "prevention" refers to a prophylactic or protective treatment of a disease or a disease condition. As used herein, the term "treatment" refers to a reduction, suppression, amelioration, or eradication of a disease condition. The "treatment" can be achieved by inhibiting the replication of viral genes or inhibiting the production of structural proteins or non-structural proteins for the production of viral particles, but is not limited thereto.

The pharmaceutical composition of the present disclosure may be formulated by using pharmaceutically acceptable carriers and/or excipients according to a method that can be easily performed by a person skilled in the art to which the present disclosure pertains, and thus the pharmaceutical composition may be provided as a unit dosage form or may be packed in a multi-dose container. In such a case, the formulation may be variously prepared into a medicine for internal use, an injection, and the like, and may be in the form of a solution in an oily or aqueous medium, a suspension, an emulsion, an extract, a pulvis, a suppository, a powder, a granule, a tablet, or a capsule, and the formulation may further contain a dispersant or a stabilizer.

In accordance with another aspect of the present disclosure, there is provided a food composition for prevention or alleviation of a virus infection, the food composition comprising rottlerin.

The food composition includes a feed composition, an auxiliary feed composition, and a feed additive.

The food composition may be used for the purpose of improving the productivity and health of a target animal, but is not limited thereto.

As used herein, the term "alleviation" refers to any action that reduces symptoms of a virus infection. The "alleviation" can be achieved by inhibiting the intracellular invasion of viruses (e.g., macropinocytosis), inhibiting the replication of viral genes, or inhibiting the production of structural proteins for the production of viral particles or non-structural proteins, but is not limited thereto.

According to still another aspect of the present disclosure, there is provided a method for treating a virus infection, the method comprising administering the above-described pharmaceutical composition to a subject.

As used herein, the term "administration" or "administer" refers to a direct administration of a therapeutically or prophylactically effective amount of the composition of the present disclosure to a subject suffering or being likely to suffer the target disease, thereby forming the same amount thereof in the body of the subject.

The term "therapeutically effective amount" of the composition refers to a content of the composition, which is sufficient to provide a therapeutic or prophylactic effect to a subject to which the composition is to be administered, and thus the term has a meaning encompassing "prophylactically effective amount".

In an embodiment of the present disclosure, examples of the subject include: mammals including humans, mice, rats, guinea pigs, dogs, cats, horses, cattle, pigs, monkeys, chimpanzees, baboons, and rhesus monkeys; and poultry, such as chickens, ducks, turkeys, and quails, but are not limited thereto.

Since the treatment method of the present disclosure comprises administering to a subject the above-described pharmaceutical composition comprising rottlerin, the overlapping description of the composition is omitted to avoid undue complexity of the present specification.

### Advantageous Effects of Invention

Features and advantages of the present disclosure are summarized as follows.
(a) The present disclosure provides a pharmaceutical composition for the prevention or treatment of a virus infection comprising rottlerin as an active ingredient.
(b) The present disclosure provides a food composition for prevention or alleviation of a virus infection comprising rottlerin.
(c) The pharmaceutical composition for the prevention or treatment of a virus infection comprising rottlerin as an active ingredient can be advantageously used to treat a virus infection caused by porcine epidemic diarrhea virus, porcine circovirus type 2, porcine reproductive and respiratory syndrome virus, or the like.

### Brief Description of Drawings

FIG. 1 shows the results of investigating cytotoxicity of an antiviral agent (microparticles).
FIG. 2 shows the results of investigating cytotoxicity of an antiviral agent (liposomes).
FIG. 3 shows an experimental design for evaluating antiviral efficacy of an antiviral agent (liposomes) according to the inoculation time.
FIG. 4 shows the results of measuring antiviral efficacy of an antiviral agent (liposomes) according to the inoculation time at 48 hours post viral infection.
FIG. 5 shows the results of measuring antiviral efficacy of an antiviral agent (liposomes) according to the inoculation concentration at 48 hours post viral infection.
FIG. 6 shows the results of investigating antiviral efficacy of an antiviral agent (liposomes) through immunostaining.
FIG. 7 shows the results of investigating the effect of an antiviral agent (liposomes) on the internalization of PRRSV through qRT-PCR.
FIG. 8 shows the animal test efficacy evaluation results of an antiviral agent (liposomes) through qRT-PCR.
FIG. 9 shows the animal test efficacy evaluation results of an antiviral agent (liposomes) through titration.
FIG. 10 shows the lung lesion score results confirmed in test groups.
FIG. 11 shows the animal test efficacy evaluation results of an antiviral agent (liposomes) through clinical symptom scores.
FIG. 12 shows the virus concentrations and antiviral agent concentrations in the tests for investigating the effects of antiviral agents on porcine epidemic diarrhea virus.
FIG. 13 shows the results of MTT assay performed to investigate the effects of antiviral agents on porcine epidemic diarrhea virus.
FIG. 14 shows the results of fluorescent immunostaining performed to investigate the effects of antiviral agents on porcine epidemic diarrhea virus.
FIG. 15 shows the results of PCR performed to investigate the effects of antiviral agents on feline coronavirus.
FIG. 16 shows the results of real-time PCR performed to investigate the effects of antiviral agents on porcine circovirus type 2.

### Mode for Carrying out the Invention

Hereinafter, the present disclosure will be described in more detail with reference to exemplary embodiments. These exemplary embodiments are provided only for the purpose of illustrating the present disclosure in more detail, and therefore, according to the purpose of the present disclosure, it would be apparent to a person skilled in the art that these examples are not construed to limit the scope of the present disclosure.

### EXAMPLES

### Preparation Example 1: Preparation of rottlerin-containing antiviral agent (microparticles)

Rottlerin used herein was classified as a rottlerin crystal sample (R0) and a rottlerin crystal-sonicated sample (R1) according to the formulation.

The rottlerin crystal sample (R0) was commercially purchased and had a purity of 95% or more.

The rottlerin crystal sample (R0) was diluted in DMSO solvent to a concentration of 10 mM, incorporated into distilled water at a ratio of 1:50 (volume ratio), and then sonicated with an amplification intensity of 40% at 0°C for 10 minutes. The prepared microparticles were centrifuged at 100,000 rpm for 1 hour, washed with distilled water to remove impurities, re-suspended, and stored at a temperature of 4°C.

### Preparation Example 2: Preparation of rottlerin-containing antiviral agent (liposomes)

Rottlerin-containing liposomes were prepared by a thin-film hydration method. More specifically, 0.86 g of hydrogenated soy phosphatidylcholine (HSPC), 0.08 g of cholesterol (phosphatidylcholine : cholesterol = 5:1 mol/mol), and 10 mg of rottlerin (rottlerin : lipid = 1:94 wt/wt) were dissolved in a methanol-chloroform solution (1:2 v/v), and then the lipid mixture was dried to obtain a thin membrane by utilizing a rotary evaporator (56°C, 252 rpm, 294 mbar; Rotavapor R-100, Buchi, USA). The lipid membrane was hydrated with PBS (62, 252 rpm) and left at room temperature for 2 hours.

The prepared liposomes were reduced to size by sonication (8s on/2s off) at an amplitude of 23% for 5 minutes using a VC505 device (Sonics & Materials, Connecticut, USA), and dialyzed using a MWCO 2000 dialysis membrane (Thermo Scientific, USA).

The prepared rottlerin-containing liposomes showed a size of 66±10 nm as a result of size measurement using dynamic light scattering (DLS) equipment, were confirmed to have a concentration of about 500 ug/ml when analyzed at a wavelength of 420 nm using a spectrometer, and were adjusted to pH of 7.4+0.1.

### Example 1: Cytotoxicity of antiviral agent (microparticles) prepared in Preparation Example 1

To investigate the cytotoxicity of the antiviral agent prepared in Preparation Example 1, water-soluble tetrazolium salt assay was performed. The rottlerin microparticles (R1) were added at various concentrations to MARC-145 cell lines and porcine alveolar macrophages, followed by incubation for one day, and then a water-soluble tetrazolium salt was added, followed by incubation for 4 hours. Thereafter, the absorbance was measured at 450 nm to determine the cell viability.

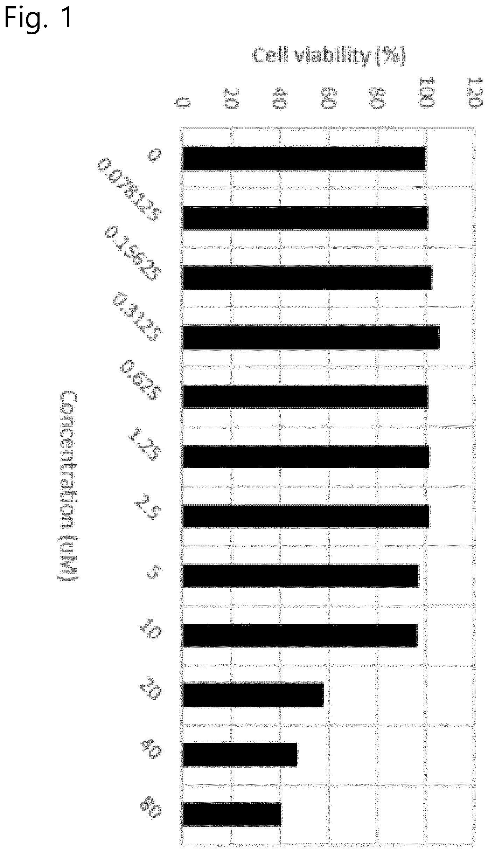

As a result of the cytotoxicity test of the antiviral agent (sonicated rottlerin microparticles, R1) used herein, the cell viability was maintained at 96.5% or more up to 10 µM (FIG. 1).

Therefore, a concentration of at most 5-10 µM was used to evaluate the efficacy of the antiviral agent of the present disclosure.

### Example 2: Cytotoxicity of antiviral agent (liposomes) prepared in Preparation Example 2

The cell viability was measured by water-soluble tetrazolium salt (WST) assay using an EZ-Cytox cell viability assay kit (Daeil Lab Service, Korea). More specifically, porcine alveolar macrophages (PAMs) and MARC-145 cells were seeded at concentrations of 1×10⁶ and 3×10⁵/ml in 96-well plates, respectively. The cells were treated with the antiviral agent (liposomes) at 0.3125, 1.25, 5, and 10 µM one to two days after seeding. The cells were incubated at 37°C and 5% CO₂ for 24 hours. Thereafter, 10 µl of EZ-Cytox was added, followed by incubation at 37°C and 5% CO₂ for 4 hours. The optical density at 450 nm was read with 620 nm as a reference to measure any background signals.

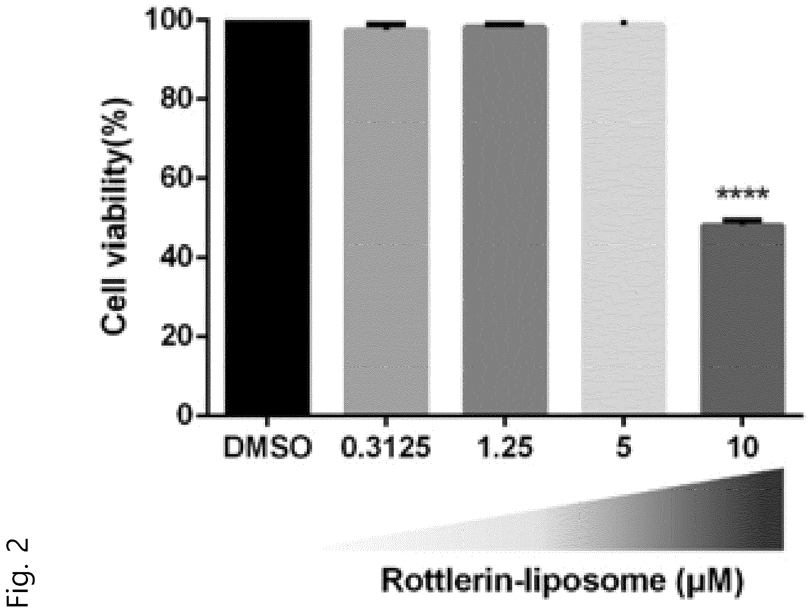

As a result of evaluating the cytotoxicity according to the concentration of rottlerin-loaded liposomes, the cytotoxicity was not shown up to 5 µM (FIG. 2).

### Example 3: Antiviral efficacy of antiviral agent (liposomes) prepared in Preparation Example 2

As for a test virus, PRRSV FL12 was used as a standard virus strain.

After 100 µL of a test virus suspension was added at a MOI of 0.1 to cell monolayers in which MARK-145 cells were incubated at a concentration of 10⁴ cells/well, the test substance was added according to the concentration and time for each test, and each test group was tested in triplicate.

Plates inoculated with PRRSV were incubated at 37°C and CO₂ for 48 hours and 72 hours. The supernatants were collected from the plates upon completion of virus incubation according to the time, and stored at -70°C. The viral titer was examined for each cell incubation supernatant in 96-well plates by using TCID₅₀.

### Example 3-1: Antiviral efficacy of antiviral agent (liposomes) according to inoculation time

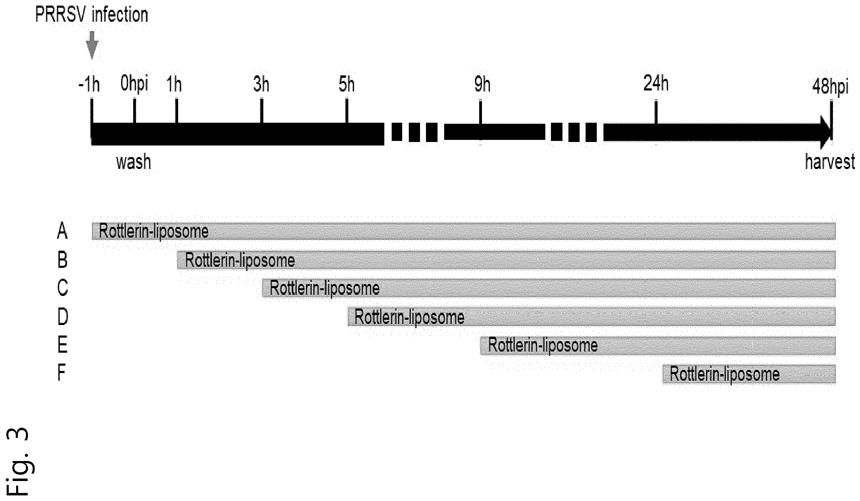

To evaluate the antiviral effect of the rottlerin liposomes according to the inoculation time, the treatment with the antiviral agent (rottlerin liposomes) was performed at 1 hour before PRRS viral infection and at 1 hour, 3 hours, 5 hours, 9 hours, and 24 hours post PRRS viral infection, and the viral titer was measured at 48 hours post viral infection (FIG. 3).

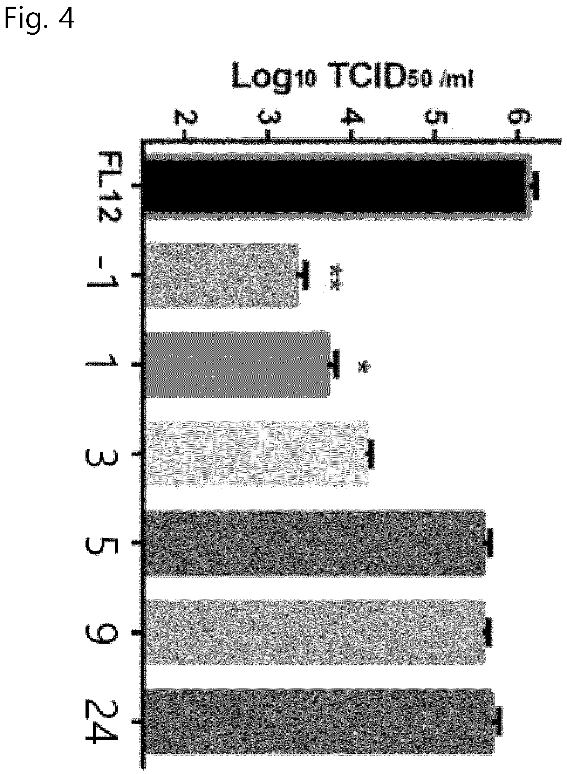

As a test result, the viral titer was reduced by 99% (2 log) or more in a group treated with rottlerin liposomes at 1 hour before PRRS viral infection and a group treated with rottlerin liposomes at 1 hour post PRRS viral infection (FIG. 4).

The above results indicate that the antiviral agent (rottlerin liposomes) of the present disclosure showed an antiviral effect and, especially, exhibited an excellent infection preventing or treating effect when used immediately before or after infection.

### Example 3-2: Antiviral efficacy of antiviral agent (liposomes) according to inoculation concentration

To evaluate the antiviral effect of rottlerin liposomes according to the inoculation concentration, the treatment with the antiviral agent (rottlerin liposomes) at 0.3125, 1.25, and 5 µM was performed to measure the viral titer at 48 hours post PRRSV infection.

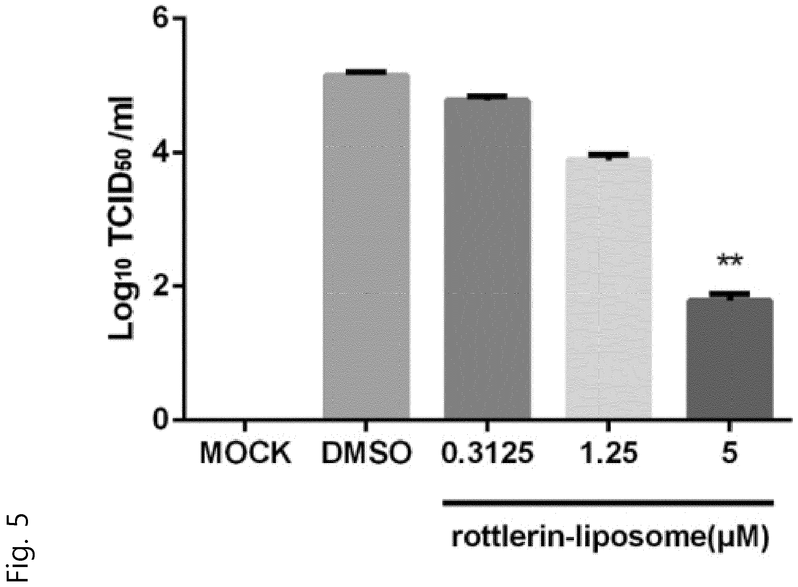

As a test result, the viral titer was reduced by 99% or more after 48 hours in a group treated with the antiviral agent at 5 µM (FIG. 5).

The above results indicate that the antiviral agent (rottlerin liposomes) of the present disclosure showed an antiviral effect and, especially, exhibited an excellent effect in a dose-dependent manner.

### Example 3-3: Antiviral efficacy of antiviral agent (liposomes) confirmed through fluorescent immunostaining

To evaluate the inhibitory effect of rottlerin or rottlerin liposomes against PRRSV protein expression, the cells were treated with 5 µM rottlerin liposomes and 10 µM rottlerin, and 48 hours after inoculation, fluorescent immunostaining was performed using primary antibody for PRRSV nucleocapsids and Alexa 488 secondary antibody.

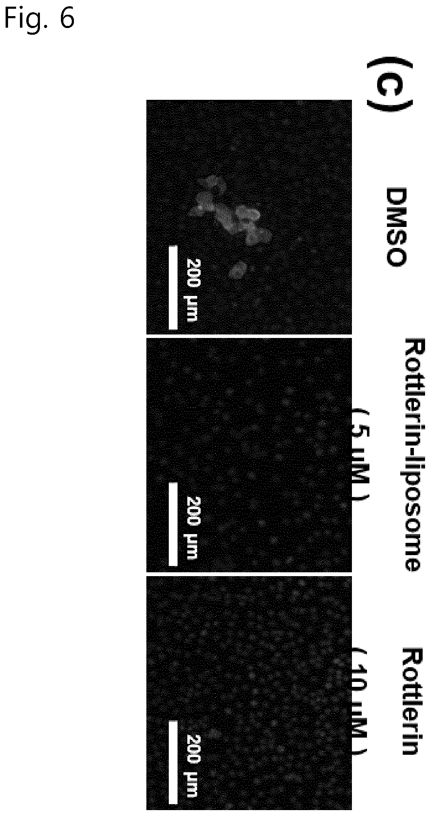

The analysis using fluorescent immunostaining confirmed that virus proteins were not detected in the cells treated with rottlerin or rottlerin-loaded liposomes (see FIG. 6).

The above results indicate that the present inventive rottlerin liposomes exhibited an excellent antiviral effect compared with typical rottlerin under the same concentration.

### Example 4: Efficacy of antiviral agent (rottlerin liposomes) prepared in Preparation Example 2 on PRRS virus internalization

### Example 4-1: Efficacy of antiviral agent (rottlerin liposomes) on PRRS virus internalization confirmed by qRT-PCR

To investigate the effect of the antiviral agent (rottlerin liposomes) on the internalization of PRRSV, an internalization test was performed.

MARC-145 cells were seeded at a concentration of 1.5 × 10⁵ cells/well in 24-well plates, followed by incubation for 2 days, and then the cells were treated with CD163 antibody or the antiviral agent (rottlerin liposomes) at 37°C for 2 hours. Thereafter, the cells were washed, infected with PRRSV FL12 at a MOI of 0.03, and treated with the antiviral agent (rottlerin liposomes) at 37°C for 1 hour. After washing, the cells were additionally treated with the antiviral agent (rottlerin liposomes) and further incubated at 37°C for 2 hours. Last, the cells were treated with proteinase K (Pro K, Takara, Korea) at 4°C for 45 minutes to remove extracellular viruses.

A group treated with CD163 antibody known as a receptor for PRRSV was used as a positive control for an antiviral effect.

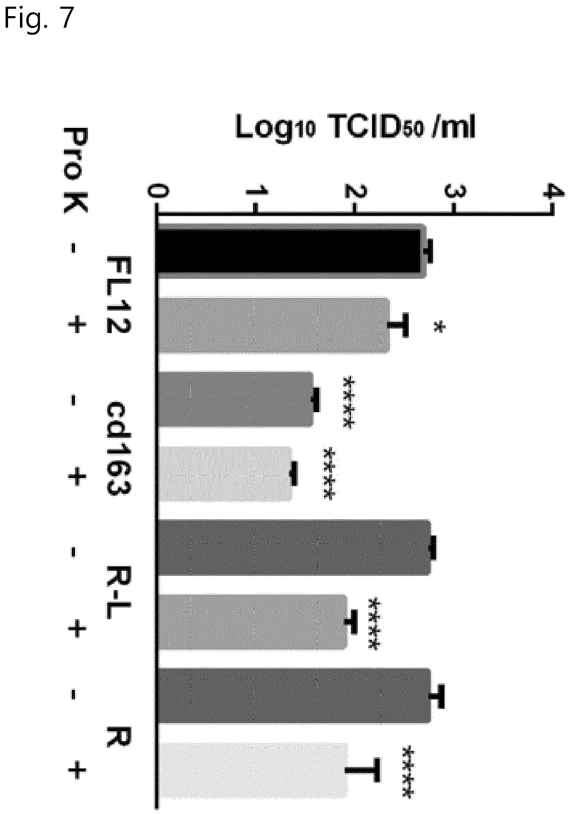

As a test result, the groups treated with rottlerin liposomes showed a lower effect than the groups treated with CD163 antibody, but they also showed a significant decrease in the intracellular viral titer (*: p<0.05, **: p<0.01, ***: P<0.001, ***: P<0.0001). (FIG. 7). The results indicate that antiviral agent of the present disclosure (rottlerin liposomes) reduced the internalization of PRRSV.

### Example 5: Efficacy of antiviral agent (rottlerin liposomes) prepared in Preparation Example 2 on PRRSV confirmed through animal model

Validity assessment for target animals were performed using 4-week-old pigs. A total of 15 pigs were used, and randomly distributed into a positive PRRSV FL12 administration group, a rottlerin liposome 100 µg administration group (R-L 100 µg), a rottlerin liposome 1 mg administration group (R-L 1 mg), and a rottlerin raw material 1 mg administration group (R 1 mg), wherein four animals were used for each group (three animals were used for the rottlerin liposome 100 µg administration group).

The pigs of each group were inoculated with 10⁵ TCID₅₀/ml FL12, and after the inoculation, 2 ml of each therapeutic agent was intranasally administered by spray.

Sera were separated from the pigs on days 1, 3, 7, 14, and 19, and the pigs were euthanized and necropsied on day 19 of inoculation. The lung tissue of each pig was collected and lesions were evaluated, thereby calculating lung lesion scores.

**TABLE 1**

| Group | FL12 | R-L 100 µg | R-L 1 mg | R 1 mg |
|---|---|---|---|---|
| Number of animals | 4 | 3 | 4 | 4 |
| Rottlerin-liposome dose | - | 100 µg | 1 mg | - |
| Rottlerin dose | - | - | - | 1 mg |
| PRRSV | 10⁵ TCID₅₀/ml | 10⁵ TCID₅₀/ml | 10⁵ TCID₅₀/ml | 10⁵ TCID₅₀/ml |

### Example 5-1: PRRSV treatment effect of antiviral agent (liposomes) confirmed through sera collected

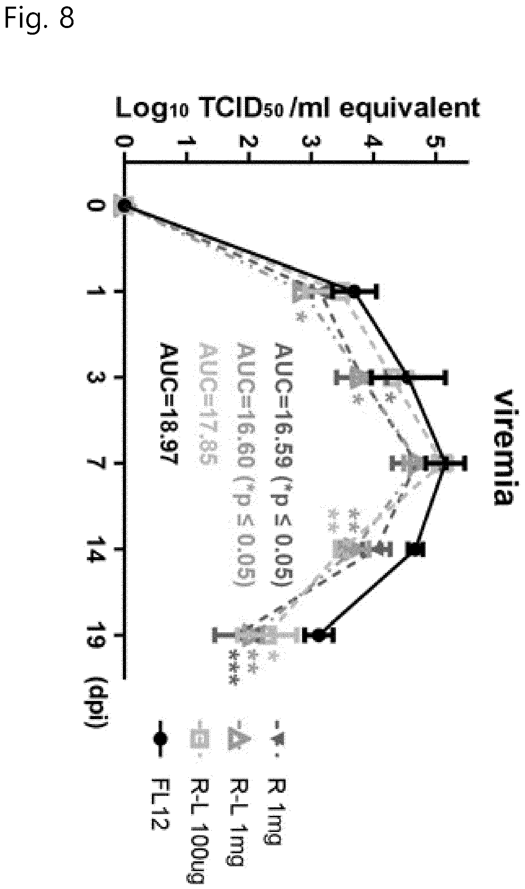

As for viremia confirmed through qRT-PCR, the rottlerin liposome (R-L) 1 mg group showed significantly lower viremia than the FL12 group except on day 7. The rottlerin liposome (R-L) 100 µg group showed significantly lower viremia than the FL12 group on days 14 and 19. When compared with the FL12 group, the areas under curve (AUC) of the rottlerin-liposome (R-L) 1 mg group and rottlerin (R) 1 mg group were significantly lower (p ≤ 0.05) (FIG. 8) .

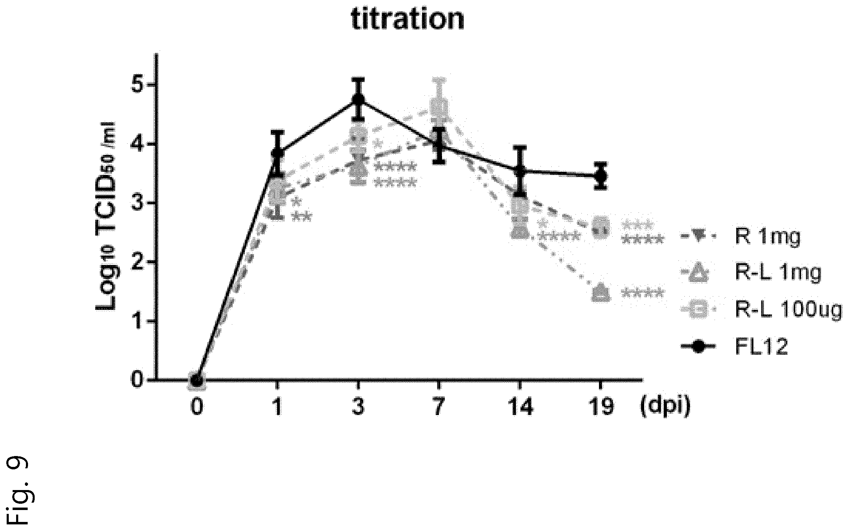

As for viremia confirmed by titration, the viremia of the rottlerin liposome (R-L) 1 mg group was significantly lower than that of the FL12 group except on day 7, and the viremia of the rottlerin liposome (R-L) 100 µg group was significantly lower than that of the FL12 group on days 3, 14, and 19 (FIG. 9).

As for porcine PRRSV antibody, there was no significant antibody difference among dates, but on day 14, all the groups showed positive for the antibody on average.

### Example 5-2: Treatment effect of antiviral agent (liposomes) confirmed through necropsy

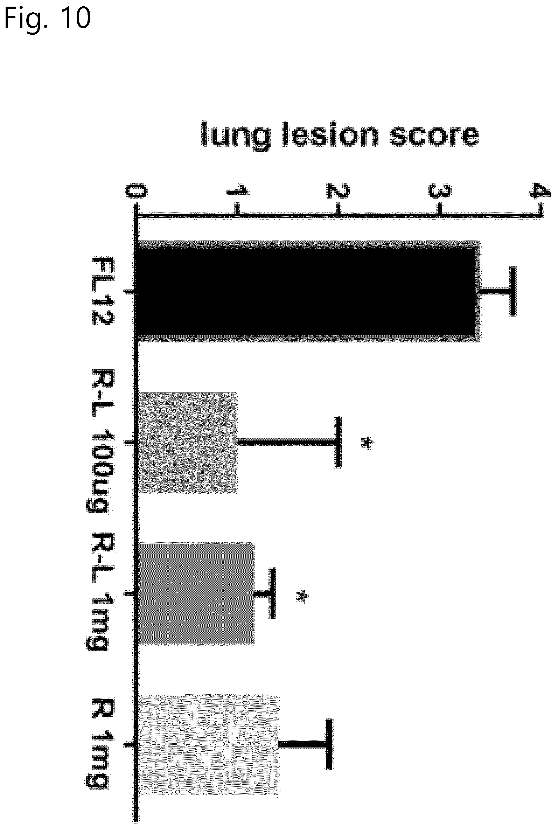

When the pigs were necropsied on day 19, the lung lesion scores were determined by visually analyzing lesions of the lung, and microscopic lung lesions were low in the rottlerin liposome (R-L) 100 µg and 1 mg groups (FIG. 10).

The lung lesion score was measured from 0 to 100 by setting respective lobes of the entire lung to the following points and scoring if there is a lesion: (ventral right anterior lobe, 5; ventral right middle lobe, 5; ventral right posterior lobe, 12.5; ventral left anterior lobe, 5; ventral left middle lobe, 5; ventral left posterior lobe, 12.5; accessory lobe, 5; dorsal right anterior lobe, 5; dorsal right middle lobe, 5; dorsal right posterior lobe, 15; dorsal left anterior lobe, 5; dorsal left middle lobe, 5; dorsal left posterior lobe, 15) .

Microscopic lung lesions were measured from 0 to 4 according to the degree of interstitial pneumonia as follows: (0, no lesions; 1, mild lesions or less than half being lesions; 2, common lesions or half being lesions; 3, moderately spread lesions or more than half being lesions; 4, severe lesions or full lesions).

### Example 5-3: Treatment effect of antiviral agent (liposomes) confirmed through observation of clinical symptoms

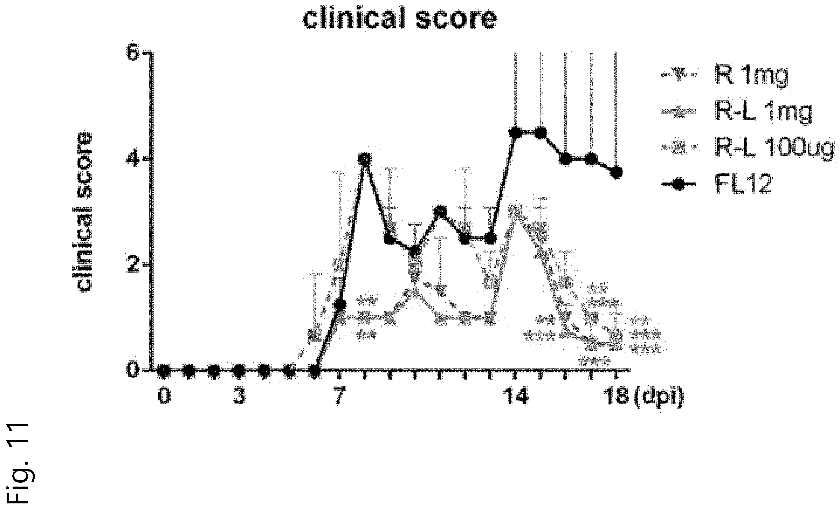

The rottlerin liposome (R-L) 100 µg group showed a significant decrease in clinical symptom score on days 17 and 18. The rottlerin liposome (R-L) 1 mg group showed a significant decrease in clinical symptom score on days 8, 16, 17, and 18. The rottlerin (R) 1 mg group showed a significant decrease in clinical symptom score on days 8, 16, 17, and 18 (FIG. 11).

Each pig was measured for clinical symptom scoring according to the following points every day: 0, normal; 1, weak dyspnea or tachypnea in the presence of stress; 2, weak dyspnea or tachypnea at rest; 3, moderate dyspnea or tachypnea in the presence of stress; 4, moderate dyspnea or tachypnea at rest; 5, severe dyspnea or tachypnea in the presence of stress; 6, severe dyspnea or tachypnea at rest.

The above results indicate that antiviral agent (liposomes) of the present invention exhibited an excellent antiviral effect.

### Example 6: Efficacy of antiviral agents (microparticles and liposomes) on porcine epidemic diarrhea (PED)

The efficacy of antiviral agents of the present disclosure on porcine epidemic diarrhea virus was investigated through MTT assay and immunostaining. SGP-M1 and SM 98 strains, which are porcine epidemic diarrhea virus, were used as test viruses.

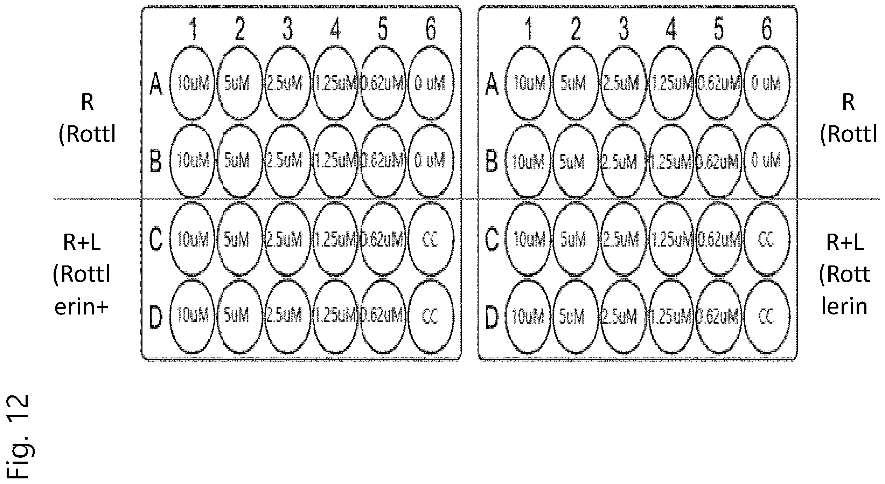

Vero cells were seeded at 2.5×10⁵ in wells and incubated for 2 days. Thereafter, 250 µL of the antiviral agents (microparticles and liposomes; 10, 5, 2.5, 1.25, 0.62, and 0 µM) and 250 µL of the test viruses SGP-M1 and SM 98 (200 TCID/well) were inoculated in the cells, and then incubated for 1 day (FIG. 12).

Then, MTT assay, real-time PCR, and fluorescent immunostaining were performed on the incubation products.

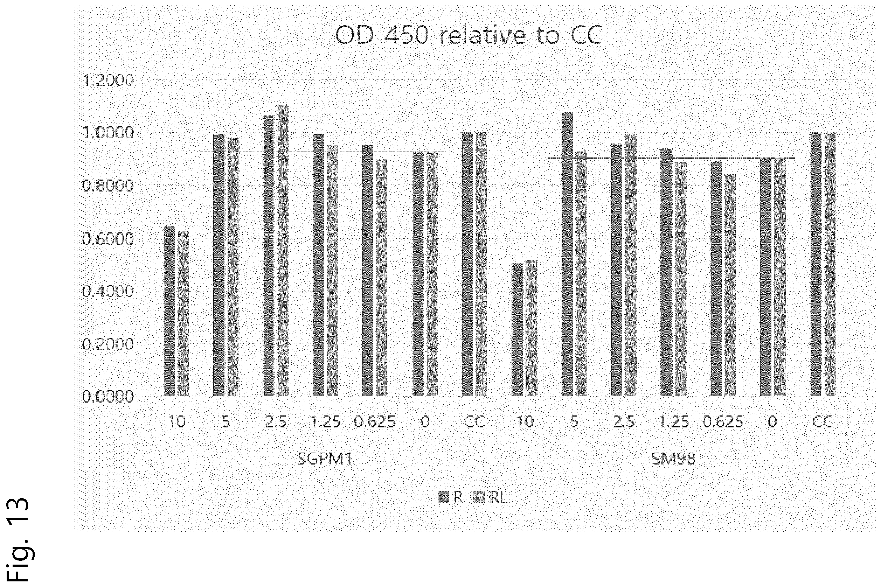

As a result of MTT assay, the SGP-M1 inoculation groups showed high cell viability when treated with the antiviral agents at 5 µM, 2.5 µM, or 1.25 µM, and the SM-98 inoculation groups showed high cell viability when treated with the antiviral agents at 5 µM or 2.5 µM (FIG. 13) .

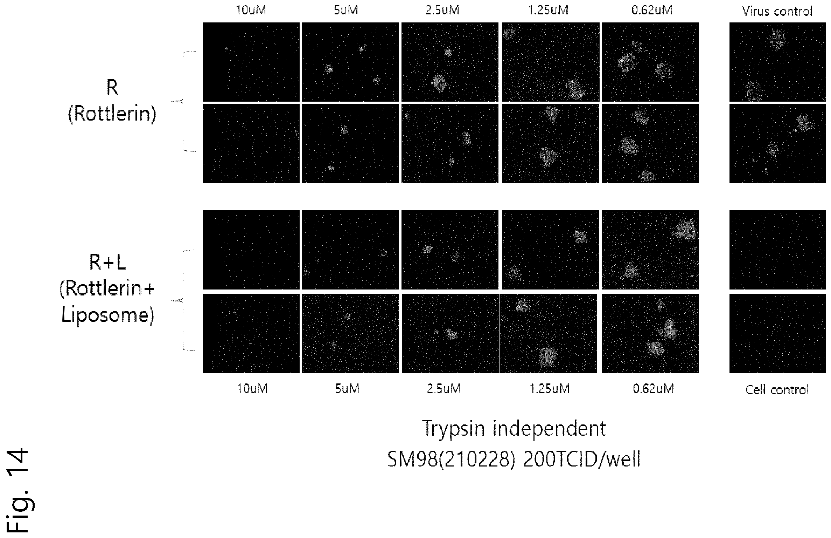

As a result of fluorescent immunostaining using anti-PEDV monoclonal IgG (Median diagnostics, Korea), the formation of virus syncytia was inhibited as the concentration of the antiviral agent increased and, especially, the treatment with the antiviral agents at 2.5 µM or more showed an excellent antiviral effect (FIG. 14).

That is, the antiviral agents (microparticles and liposomes) showed an antiviral effect on porcine epidemic diarrhea virus and, especially, showed an excellent effect at a concentration of 5 µM or 2.5 µM.

### Example 7: Efficacy of antiviral agent (liposomes) on feline coronavirus

The effect of a combination of the present inventive composition and remdesivir on feline coronavirus was investigated through RT-PCR. A specific test method was as follows.

Viral RNA was extracted from sera by using the Patho Gene-spin^{™} DNA/RNA Extraction Kit (Intron Bio, Korea), and RT-PCR diagnosis was performed using Maxime^{™} RT-PCR PreMix (Intron bio, Korea). The primers used in RT-PCR were (5'-ACG GTG TCT GGG TTG CAA G-3', SEQ ID NO: 4) and FCoV-R (5'-GGC TAT GAT TGT ATC CTC AAC AT-3', SEQ ID NO: 5), and the test was performed according to the method of the kit manufacturer. RT-PCR conditions were: 45°C for 30 minutes and 95°C for 5 minutes, and then 35 cycles of 95°C for 30 seconds, 52°C for 30 seconds, and 72°C for 45 seconds, followed by incubation at 72°C for 7 minutes. The PCR products were subjected to electrophoresis on 2% agarose gels to confirm bands.

The cats confirmed to be positive in the diagnosis of feline infectious peritonitis (FIP) by the method as above were intranasally administered 0.3 ml of a liposome agent (rottlerin 1 mg/ml and remdesivir 1 mg/ml) and intraperitoneally administered 1 ml of the liposome agent. Each injection was performed at the intervals of 3 days, and blood was collected before injection to determine the presence or absence of viruses.

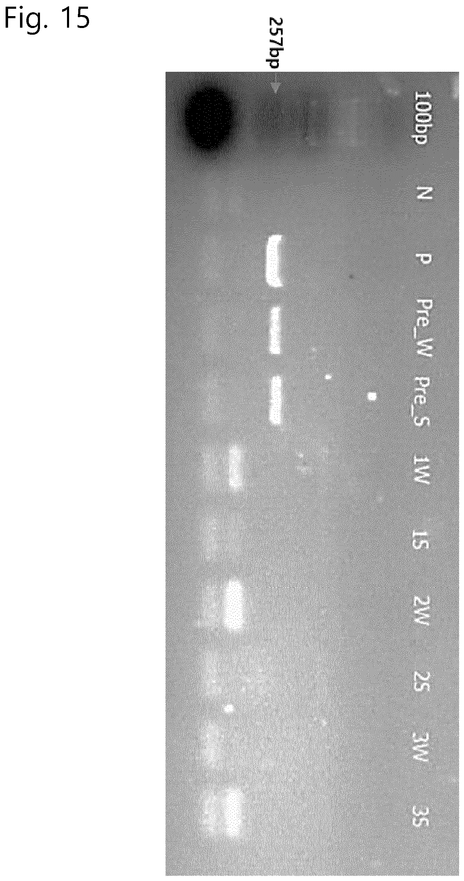

The cats initially confirmed to have positive bands for FIP in the whole blood and serum showed no viral DNA detection in both whole blood and serum on day 3 after administration of the primary therapeutic agent. Even on day 9 after administration of the secondary and tertiary therapeutic agents, viral DNA was not continuously detected, and after administration of the secondary therapeutic agent, clinical symptoms disappeared, spontaneous easting was recovered, and diarrhea lasting until day 5 were recovered (FIG. 15).

Respective symbols in FIG. 15 represent the followings: N: negative control, P: positive control, Pre_W: whole blood before administration of therapeutic agent, Pre_S: serum before administration of therapeutic agent, 1W: whole blood 3 days after administration of primary therapeutic agent, 1S: serum 3 days after administration of primary therapeutic agent, 2W: whole blood 3 days after administration of secondary therapeutic agent, 2S: serum 3 days after administration of secondary therapeutic agent, 3W: whole blood 3 days after administration of tertiary therapeutic agent, 3S: serum 3 days after administration of tertiary therapeutic agent.

It can be therefore seen that the composition comprising rottlerin of the present disclosure showed an excellent antiviral effect even on feline infectious peritonitis virus.

### Example 8: Efficacy of antiviral agent (liposomes) on porcine circovirus type 2

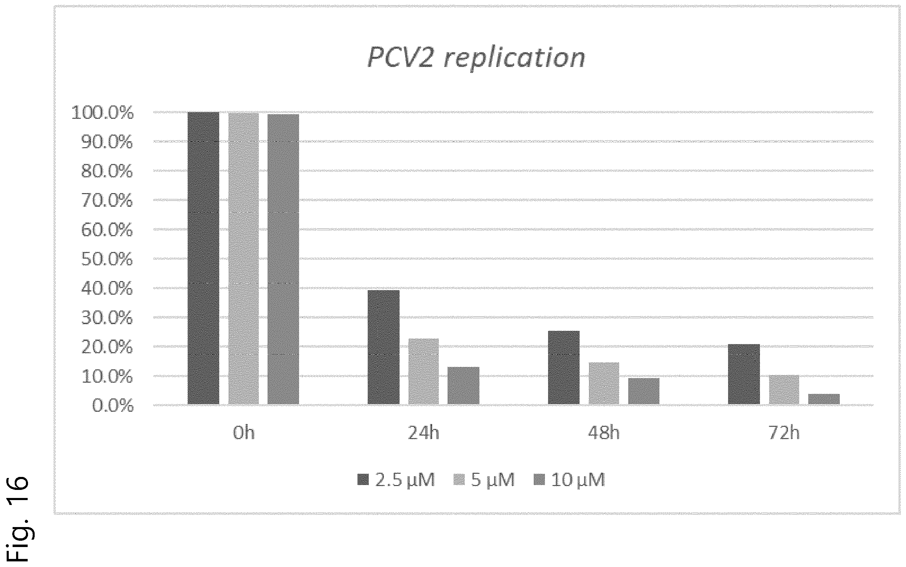

To evaluate the efficacy of the present inventive antiviral agent (liposomes) on porcine circovirus type 2, PCV2 isolated from lymph nodes of PMWS-infected pigs in a Korean farm were incubated with PK-15 cells. Specifically, PK-15 cells were incubated in a culture flask with a floor area of 175 cm², and then 5 ml of PCV2 was inoculated with 10⁷ TCID (by PCR) per ml, followed by addition of 50 ml of DMEM containing 1.5% FBS, and then the cells were incubated at 37°C and 5 % CO₂ for 5 days. A group inoculated with only the virus was used as a positive control, and test groups inoculated with the virus and rottlerin liposomes (2.5 µM, 5 µM, and 10 µM) were prepared, and then, the genome copy number of the virus was measured through real-time PCR at 0, 12, 24, 48, and 72 hours after inoculation. The results are shown in Table 2 and FIG. 16.

As shown Table 2 and FIG. 16, the genome copy number of PCV2 was reduced as the concentration of rottlerin liposomes was higher. It can be therefore seen that the composition comprising rottlerin of the present disclosure showed an excellent antiviral effect even on PCV2.

**TABLE 2**

| Conc. of Rottlerin liposome (µM) | Viral DNA copy (%) | | | |
|---|---|---|---|---|
| | 0h | 24h | 48h | 72h |
| 2.5 | 100.0 | 39.3 | 25.5 | 21.0 |
| 5 | 99.6 | 22.9 | 14.6 | 10.5 |
| 10 | 99.1 | 13.2 | 9.3 | 4.1 |

Although the present disclosure has described in detail with reference to the specific features, it will be apparent to those skilled in the art that this description is only for a preferred embodiment and does not limit the scope of the present disclosure.

## Claims

1. A pharmaceutical composition for prevention or treatment of a virus infection, the pharmaceutical composition comprising rottlerin as an active ingredient.

2. The pharmaceutical composition according to claim 1, wherein the rottlerin is in the form of microparticles.

3. The pharmaceutical composition according to claim 1, wherein the rottlerin is contained in liposomes composed of a lipid.

4. The pharmaceutical composition according to claim 3, wherein the lipid comprises a phosphatidylcholine and a sterol-based compound.

5. The pharmaceutical composition according to claim 4, wherein the phosphatidylcholine and the sterol-based compound have a molar ratio of 0.1-15:1.

6. The pharmaceutical composition according to claim 3, wherein the weight ratio of the lipid and the rottlerin is 0.1-150:1.

7. The pharmaceutical composition according to claim 4, wherein the phosphatidylcholine is selected from the group consisting of hydrogenated soy phosphatidylcholine (HSPC), 1,2-dimyristoyl-sn-glycero-3-phosphocholine (DMPC), 1,2-dihexanoyl-sn-glycero-3-phosphocholine (DHPC), 1,2-diheptanoyl-sn-glycero-3-phosphocholine, 1,2-dioctanoyl-sn-glycero-3-phosphocholine, 1,2-dinonanoyl-sn-glycero-3-phosphocholine, 1,2-didecanoyl-sn-glycero-3-phosphocholine, 1,2-diundecanoyl-sn-glycero-3-phosphocholine, 1,2-dilauroyl-sn-glycero-3-phosphocholine (DLPC), 1,2-ditridecanoyl-sn-glycero-3-phosphocholine, 1,2-dipentadecanoyl-sn-glycero-3-phosphocholine, 1,2-dipalmitoyl-sn-glycero-3-phosphocholine (DPPC), 1,2-diheptadecanoyl-sn-glycero-3-phosphocholine, and 1,2-distearoyl-sn-glycero-3-phosphocholine (DSPC).

8. The pharmaceutical composition according to claim 4, wherein the sterol-based compound is selected from the group consisting of cholesterol, 3b-[N-(N',N'-dimethylaminoethane)-cabamyl]cholesterol (DC-Chol), stigmasterol, campesterol, sitosterol, ergosterol, lanosterol, dinosterol, gorgosterol, avenasterol, saringosterol, fucosterol, cholesteryl hemisuccinate, cholesteryl benzoate, cholesteryl oleate, cholesteryl oleyl carbonate, cholesteryl isostearate, cholesteryl linoleate, cholesteryl acetate, cholesteryl palmitate, cholesteryl stearate, cholesteryl chloride, cholesteryl nonanoate, and cholesteryl arachidonate.

9. The pharmaceutical composition according to claim 1, wherein the virus belongs to a family selected from the group consisting of the families Bunyaviridae, Arteriviridae, Coronaviridae, Circoviridae, Filoviridae, Flaviviridae, Hepadnaviridae, Herpesviridae, Orthomyxoviridae, Poxviridae, Rhabdoviridae, Retroviridae, Togaviridae, and Herpesviridae.

10. The pharmaceutical composition according to claim 1, wherein the virus is porcine reproductive and respiratory syndrome virus or porcine circovirus type 2.

11. The pharmaceutical composition according to claim 1, wherein the virus is a coronavirus selected from the group consisting of porcine epidemic diarrhea virus (PEDV), transmissible gastroenteritis virus (TGEV), porcine hemagglutinating encephalomyelitis virus (PHEV), porcine deltacoronavirus (PDCoV), canine coronavirus (CCoV), feline coronavirus (FCoV), bovine coronavirus (BCoV), equine coronavirus (EqCoV), infectious bronchitis virus (IBV), and murine coronavirus (MuCoV).

12. The pharmaceutical composition according to claim 1, wherein the pharmaceutical composition is administered through an intranasal administration, an oral administration, or a combination thereof.

13. A food composition for prevention or alleviation of a virus infection, the food composition comprising rottlerin.

14. A method for treating a virus infection, the method comprising administering the pharmaceutical composition according to claim 1 to a subject.
